# EUROPEAN PATENT SPECIFICATION

(11) **EP 3 669 851 B1**
(45) Date of publication and mention of the grant of the patent: **27.10.2021**
(21) Application number: 19215913.5
(22) Date of filing: 13.12.2019
(51) Int. Cl.: A61J 1/06, A61F 9/00, A61J 1/14

(54) **PLURAL-USE DROP-BY-DROP DISPENSER FOR STERILE LIQUID PRODUCT, PARTICULARLY FOR COLLYRIUM OR OTHER MEDICINAL PRODUCT OR MEDICAL DEVICE**
MEHRWEGTRÖPFCHENSPENDER FÜR STERILES FLÜSSIGPRODUKT, INSBESONDERE FÜR COLLYRIUM ODER ANDERES MEDIZINISCHES PRODUKT ODER MEDIZINISCHE VORRICHTUNG
DISTRIBUTEUR GOUTTE À GOUTTE À USAGE MULTIPLE POUR PRODUIT LIQUIDE STÉRILE, EN PARTICULIER POUR COLLYRE OU AUTRE PRODUIT MÉDICINAL OU DISPOSITIF MÉDICAL

(30) Priority: 21.12.2018 IT 201800020839
(43) Date of publication of application: 24.06.2020
(73) Proprietor: Paolo Gobbi Frattini S.r.l., 20135 Milano (IT); Giannetto, Antonino Claudio, 95123 Catania (IT); ARIM FDF S.R.L., 20145 Milano (IT)
(72) Inventor: GOBBI FRATTINI, Paolo Giuseppe, 23035 Sondalo, SO (IT)
(74) Representative: Mittler, Enrico

(56) References cited:
- EP-A2- 1 266 840
- WO-A1-93/10015
- IT-A1- UA20 161 615

## Description

The present invention relates to a plural-use drop-by-drop dispenser for sterile liquid product, particularly for collyrium or other medicinal product or medical device.

Plural-use drop-by-drop dispensers for liquid medicinal products, in particular for collyrium for caring ocular diseases, are very common in the pharmaceutical field.

One of these is for example, described in Italian Patent Application No. 102016000026325 filed on 14 March 2016, and comprises a vial for containment of liquid product, a valve device which is normally hermetically closed by an elastically deformable closing element which can be opened by axial external push for controlled drop-by-drop dispensing of the liquid product contained inside the vial and successively is automatically hermetically reclosable at the stop of said external push, and a cartridge with tubular tang and sterilizing filter which can be handled so that the aforesaid tubular tang can be pushed from outside against said elastically deformable element for its opening for drop-by-drop dispensing of the liquid product and can be successively extracted from said elastically deformable element for its hermetic reclosure at the end of dispensing.

The main problem of this known dispenser consists of the fact that once dispensing has terminated, a small residue of product may remain outside the filter, which may be contaminated by the outside air and in turn contaminate the medicinal product at the successive dispensing. The same outside air may be a source of contamination also in the absence of residue of medicinal product.

It is the object of the present invention to make a dispenser of the aforesaid type which does not have the above-mentioned problem.

According to the invention, such an object is achieved with a plural-use drop-by-drop dispenser for sterile liquid product, particularly for medicinal product, even more specifically for dispensing collyrium, comprising a vial for containment of the liquid product and a connector provided with a valve device which is normally hermetically closed by an elastically deformable closing element which can be opened by axial external push for controlled drop-by-drop dispensing of the liquid product contained inside the vial and is successively automatically hermetically reclosable at the stop of said external push, and a cartridge with tubular tang and sterilizing filter which can be handled so that the aforesaid tubular tang can be pushed from the outside against said elastically deformable element for its opening for drop-by-drop dispensing of the liquid product and can be successively extracted from said elastically deformable element for its hermetic reclosure at the end of dispensing, characterized in that said cartridge is provided with an end filter-holding bottom element provided with a drop-by-drop dispensing spout, possibly in antimicrobic material, with elastically flexible converging lips, which open under pressure of the liquid product during dispensing operation and elastically hermetically reclose at the end of the dispensing operation.

A removable protecting cover, possibly in antimicrobic material, completely covers the end bottom element of the filter and the aforesaid dispensing spout, thus keeping the two lips of the spout tightly closed between one dispensing operation and the other.

The converging lips of the dispensing spout in essence form a check valve which keeps the area downstream of the cartridge filter sterile and simultaneously prevents the return of liquid residues and/or air therein, thus avoiding any possibility of contamination of the product at the successive dispensing operation.

A preferred embodiment of the dispenser according to the invention is now described by way of non-limiting example with reference to the accompanying drawings, in which:
figure 1 shows a dispenser according to the invention, in closed condition between one dispensing operation and the successive, with protecting cover applied;
figure 2 shows the same dispenser, again in closed position, but with protecting cover removed;
figure 3 shows the same dispenser in dispensing operation of the liquid product.

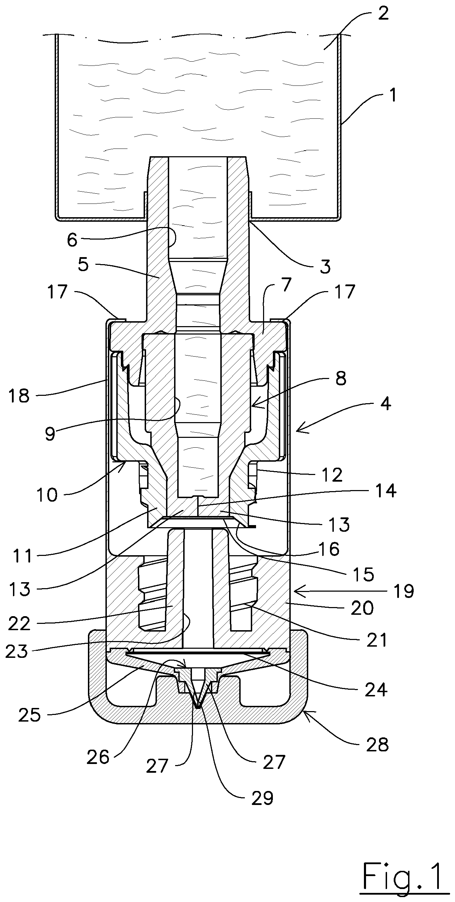

A vial for containment of a medicinal liquid product is indicated in figure 1 with 1, the medicinal liquid product being e.g. collyrium, which in turn in indicated with 2. Vial 1 preferably is made in flexible material so as to allow the user to exert pressure to push the liquid product towards an outlet mouth 3 of the vial.

A connector 4 is forced and blocked in mouth 3, the connector comprising a main body 5 crossed by an axial bore 6, a widened collar 7 and a valve device consisting of a closure plug 8 in elastically deformable plastic material, which in turn is crossed by an axial bore 9 arranged to extend bore 6. The closing plug 8 is abutted against the base of collar 7 and is kept in such a position by a ring nut 10 screwed to collar 7. The ring nut 10 has an elastically deformable narrow end mouth 11 provided with external threading 12.

The closing plug 8 ends at the bottom with a pair of elastically flexible lips 13 separated by a thin axial slit 14 having rectangular section, which ends immediately before the lower extremity of plug 8.

A thin elastic sealing membrane 15 formed in one piece with plug 8 is arranged immediately below the two flexible lips 13, at the beginning of a conical flaring 16 of the end mouth 11 of the ring nut 10.

The structural features of membrane 15 are those described in EP 2 667 839 B1, to which explicit descriptive reference is made herein.

Two folds 17 of an axial extension 18 of a filter-holding cartridge 19, which is axially slidable with respect to connector 4, rest on collar 7. Cartridge 19 is formed by an external body 20 provided with an internal threading 21 compatible for screw coupling with the external threading 12 of the end mouth 11 of the ring nut 10.

An internal tubular tang 22 with axial bore 23, which projects upwards with respect to the external body 20, extends from the base of the external body 20 of cartridge 19.

A sterilizing filter 24 carried by a cup-shaped end bottom element 25 hooked to the base of body 20 is positioned below the body 20 of cartridge 19.

The bottom element 25 is provided with a dispensing spout 26, possibly in antimicrobic material, which has elastically flexible converging lips 27, which are normally hermetically closed, which are capable of opening under pressure of the liquid product for dispensing successive drops of the liquid product 2 and of elastically hermetically reclosing at the end of the dispensing operation.

A removable protecting cover 28, possibly in antimicrobic material, completely covers the end bottom element 25 of cartridge 19 and the aforesaid dispensing spout 26. A conical recess 29 thereof houses the two lips 27 of spout 26, keeping them tightly closed between one dispensing operation and the other.

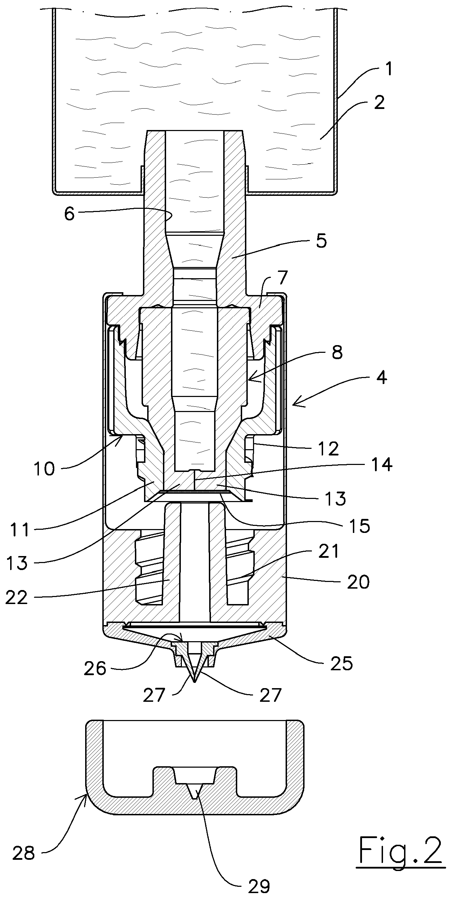

In order to proceed with dispensing a desired number of drops of the liquid product 2, firstly there is a need to remove cover 28, as shown in figure 2.

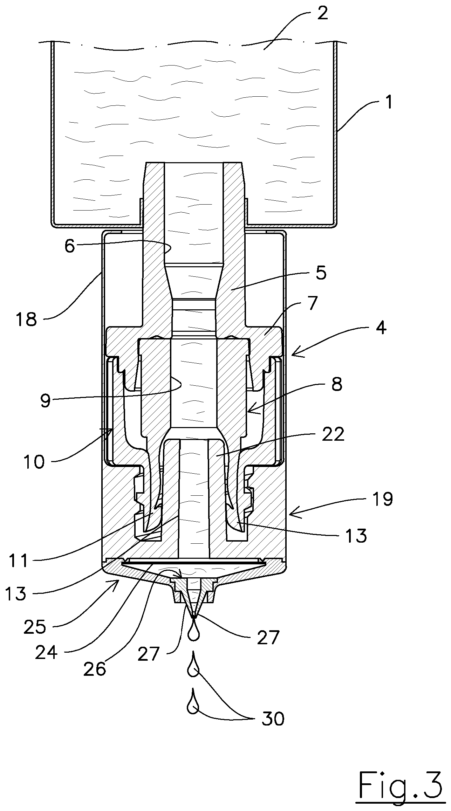

Successively, cartridge 19 is raised and screwed onto the end mouth 11 of the ring nut 10. By doing this, the internal tang 22 of cartridge 19 is push inserted into plug 8, thus first generating the temporary break of membrane 15, and then the temporary moving away of the flexible lips 13 with subsequent widening of slit 14 and the aligned holes 6, 9 and 23 being put into communication, as shown in figure 3.

Liquid 2, possibly helped by the pressure of the user's hand on the wall of vial 1, may descend along bore 23, pass through filter 24 and finally come out, in the shape of drops 30, from the space created between the flexible lips 27 and spout 26.

Once the desired number of drops is dispensed, the assembly is turned over, with subsequent return of the liquid product into vial 1 and automatic hermetic reclosure of the lips 27 of spout 26, and cartridge 19 is unscrewed and moved away from the closure plug 8, thus allowing the hermetic reclosure of the flexible lips 13 and of membrane 15. No contamination from the outside is now possible due to the hermetic closure ensured by spout 26.

Finally, the protecting cover 28 is applied again and the assembly is now ready for future dispensing operations in complete safety.

## Claims

1. Plural-use drop-by-drop dispenser for sterile liquid product, particularly for collyrium or other medicinal product, comprising a vial (1) for containment of the liquid product (2) and a connector (4) provided with a valve device (8) which is normally hermetically closed by an elastically deformable closing element (13-15) which can be opened by axial external push for controlled drop-by-drop dispensing of the liquid product (2) contained inside the vial (1) and is successively automatically hermetically reclosable at the stop of said external push, and a cartridge (19) with tubular tang (22) and sterilizing filter (24) which can be handled so that said tubular tang (22) can be pushed from outside against said elastically deformable element (13-15) for its opening for drop-by-drop dispensing of the liquid product and can be successively extracted from said elastically deformable element (13-15) for its hermetic reclosure at the end of dispensing, **characterized in that** said cartridge (19) is provided with an end filter-holding bottom element (25) provided with a drop-by-drop dispensing spout, possibly in antimicrobic material, with elastically flexible converging lips (27), which open under pressure of the liquid product (2) during dispensing operation and automatically elastically hermetically reclose at the end of the dispensing operation.

2. Dispenser according to claim 1, wherein said cartridge (19) comprises an external body (20), which is axially slidable and screwable on a part (11) of said connector (4), and said tubular tang (22) extends inside said external body (20) and projects axially therefrom and towards said connector (4) so that axial sliding and screwing of the cartridge (19) on said part (11) of the connector (4) causes opening of said elastically deformable element (13-15) by axial push from said tang (22).

3. Dispenser according to claim 2, wherein said valve device (8) is formed by a closure plug (8) of the connector (4) which is passed through by an axial bore (9), and said elastically deformable element (13-15) is placed to close said axial bore (9) and comprises a pair of elastically flexible lips (13), which are normally closed to define a thin slit (14) between them and an elastic sealing membrane (15) placed to close said slit (14) and are openable together with said flexible lips (13) by push of said tubular tang (22) of the cartridge (19), said flexible lips (13) and said membrane (15) being automatically reclosable to hermetic condition upon extraction of said tubular tang (22).

4. Dispenser according to claim 1, further comprising a removable protecting cover (28), possibly in antimicrobic material, placed to cover said end bottom element (25) of the cartridge (19) and of said dispensing spout (26).

5. Dispenser according to claim 1, wherein said protecting cover (28) is provided with a conical recess (29) intended to accommodate and press together said flexible lips (27) of the spout (26) when said protecting cover is applied to cover said bottom element (25) and said spout (26).

## Patentansprüche

1. Mehrweg-Tropfenspender für ein steriles flüssiges Produkt, insbesondere für Kollyrium oder ein anderes medizinisches Produkt, umfassend eine Durchstechflasche (1) zur Aufnahme des flüssigen Produkts (2) und ein Verbindungsstück (4) mit einer Ventilvorrichtung (8), die normalerweise durch ein elastisch verformbares Verschlusselement (13-15) hermetisch verschlossen ist, das durch axialen Außendruck zur kontrollierten tropfenweisen Abgabe des im Inneren der Durchstechflasche (1) enthaltenen flüssigen Produkts (2) geöffnet werden kann und bei der Beendigung des Außendrucks sukzessive automatisch hermetisch wiederverschließbar ist, und einer Kartusche (19) mit einem Rohrstutzen (22) und einem Sterilisationsfilter (24), die so gehandhabt werden kann, dass der Rohrstutzen (22) von außen gegen das elastisch verformbare Element (13-15) für dessen Öffnen zur tropfenweisen Abgabe des flüssigen Produkts gedrückt werden kann und sukzessive aus dem elastisch verformbaren Element (13-15) für dessen hermetisches Wiederverschließen am Ende der Abgabe herausgezogen werden kann, **dadurch gekennzeichnet, dass** die Kartusche (19) mit einem Endfilter-Halte-Bodenelement (25) versehen ist, das mit einer Tülle zur tropfenweisen Abgabe, gegebenenfalls aus antimikrobiellem Material, mit elastisch flexiblen aufeinander zulaufenden Lippen (27), die sich unter Druck des flüssigen Produkts (2) während einer Abgabebetätigung öffnen und sich am Ende der Abgabebetätigung automatisch elastisch hermetisch wiederverschließen, versehen ist.

2. Spender nach Anspruch 1, wobei die Kartusche (19) einen Außenkörper (20) umfasst, der axial verschiebbar und auf ein Teil (11) des Verbindungsstücks (4) schraubbar ist, und wobei sich der Rohrstutzen (22) in dem Außenkörper (20) erstreckt und axial davon und in Richtung des Verbindungsstücks (4) vorsteht, so dass ein axiales Verschieben und Schrauben der Kartusche (19) auf das Teil (11) des Verbindungsstücks (4) das Öffnen des elastisch verformbaren Elements (13-15) durch axiales Drücken durch den Rohrstutzen (22) bewirkt.

3. Spender nach Anspruch 2, wobei die Ventilvorrichtung (8) durch einen Schließstopfen (8) des Verbindungsstücks (4) ausgebildet wird, der von einer axialen Bohrung (9) durchlaufen wird, und das elastisch verformbare Element (13-15) platziert ist, um die axiale Bohrung (9) zu schließen, und ein Paar elastisch flexibler Lippen (13), die normalerweise geschlossen sind, um einen dünnen Schlitz (14) zwischen ihnen zu definieren, und eine elastischen Dichtungsmembran (15), die platziert ist, um den Schlitz (14) zu schließen, umfasst und zusammen mit den flexiblen Lippen (13) durch Drücken des Rohrstutzens (22) der Kartusche (19) öffenbar sind, wobei die flexiblen Lippen (13) und die Membran (15) für einen hermetischen Zustand mit Herausziehen des Rohrstutzens (22) automatisch wiederverschließbar sind.

4. Spender nach Anspruch 1, ferner umfassend eine entfernbare Schutzabdeckung (28), gegebenenfalls aus antimikrobiellem Material, die platziert ist, um das Endbodenelement (25) der Kartusche (19) und die Abgabetülle (26) abzudecken.

5. Spender nach Anspruch 1, wobei die Schutzabdeckung (28) mit einer konischen Aussparung (29) versehen ist, die dazu gedacht ist, die flexiblen Lippen (27) der Tülle (26) aufzunehmen und zusammenzudrücken, wenn die Schutzabdeckung aufgebracht wird, um das Bodenelement (25) und die Tülle (26) abzudecken.

## Revendications

1. Distributeur goutte à goutte à usages multiples pour produit liquide stérile, notamment pour collyre ou autre produit médicinal, comprenant un flacon (1) pour contenir le produit liquide (2) et un connecteur (4) muni d'un dispositif de vanne (8) qui est normalement fermé hermétiquement par un élément de fermeture élastiquement déformable (13-15) qui peut être ouvert par poussée externe axiale pour la distribution goutte à goutte contrôlée du produit liquide (2) contenu à l'intérieur du flacon (1) et est successivement automatiquement refermable hermétiquement à l'arrêt de ladite poussée externe, et une cartouche (19) avec un tenon tubulaire (22) et un filtre stérilisant (24) qui peut être manipulée de telle sorte que ledit tenon tubulaire (22) peut être poussé de l'extérieur contre ledit élément élastiquement déformable (13-15) pour son ouverture pour la distribution goutte à goutte du produit liquide et peut être successivement extrait dudit élément élastiquement déformable (13-15) pour sa refermeture hermétique à la fin de la distribution, **caractérisé en ce que** ladite cartouche (19) est munie d'un élément inférieur (25) d'extrémité porte-filtre muni d'un bec de distribution goutte à goutte, éventuellement en matériau antimicrobien, avec des lèvres convergentes élastiquement flexibles (27), qui s'ouvrent sous la pression du produit liquide (2) lors de l'opération de distribution et se referment hermétiquement élastiquement automatiquement à la fin de l'opération de distribution.

2. Distributeur selon la revendication 1, dans lequel ladite cartouche (19) comprend un corps externe (20), qui est axialement coulissant et vissable sur une partie (11) dudit connecteur (4), et ledit tenon tubulaire (22) s'étend à l'intérieur dudit corps externe (20) et fait saillie axialement de celui-ci et vers ledit connecteur (4) de sorte que le coulissement axial et le vissage de la cartouche (19) sur ladite partie (11) du connecteur (4) provoquent l'ouverture dudit élément élastiquement déformable (13-15) par poussée axiale à partir dudit tenon (22).

3. Distributeur selon la revendication 2, dans lequel ledit dispositif de vanne (8) est formé par un bouchon de fermeture (8) du connecteur (4) qui est traversé par un alésage axial (9), et ledit élément élastiquement déformable (13-15) est placé pour fermer ledit alésage axial (9) et comprend une paire de lèvres élastiquement flexibles (13), qui sont normalement fermées pour définir une mince fente (14) entre elles et une membrane d'étanchéité élastique (15) placée pour fermer ladite fente (14) et peuvent être ouvertes conjointement avec lesdites lèvres flexibles (13) par poussée dudit tenon tubulaire (22) de la cartouche (19), lesdites lèvres flexibles (13) et ladite membrane (15) pouvant être refermées automatiquement à l'état hermétique lors de l'extraction dudit tenon tubulaire (22).

4. Distributeur selon la revendication 1, comprenant en outre un couvercle de protection amovible (28), éventuellement en matériau antimicrobien, placé pour couvrir ledit élément inférieur d'extrémité (25) de la cartouche (19) et ledit bec de distribution (26).

5. Distributeur selon la revendication 1, dans lequel ledit couvercle de protection (28) est muni d'un évidement conique (29) destiné à recevoir et à presser ensemble lesdites lèvres flexibles (27) du bec (26) lorsque ledit couvercle de protection est appliqué pour couvrir ledit élément inférieur (25) et ledit bec (26).
